# DEMANDE DE BREVET EUROPEEN

(11) **EP 1 541 128 A2**
(43) Date de publication de la demande: **15.06.2005**
(21) Numéro de dépôt: 04293055.2
(22) Date de dépôt: 26.11.1998
(51) Int. Cl.: A61K 7/48

(54) **Composition cosmétique contenant des dérivés de l'acide cinnamique et leur utilisation**

(30) Priorité: 19.12.1997 FR 9716180
(62) Demande divisionnaire de: 98402959.5
(71) Demandeur: L'OREAL, 75008 Paris (FR)
(72) Inventeur: Breton, Lionel, 78000 Versailles (FR); Girerd-Dugue, Florence, 34130 Maugio (FR); Renault, Béatrice, 94410 Saint Maurice (FR)
(74) Mandataire: Allab, Myriam

(57) **Abrégé**

L'invention se rapporte à l'utilisation d'un dérivé d'acide cinnamique dans une composition cosmétique en tant qu'agent raffermissant de la peau et/ou des muqueuses.

L'invention a également pour objet une composition raffermissante comprenant un dérivé d'acide cinnamique.

## Description

L'invention se rapporte à l'utilisation de l'acide cinnamique ou d'au moins un de ses dérivés dans une composition cosmétique raffermissante. En particulier, les compositions de l'invention sont destinées à stimuler la restructuration de la peau et/ou des muqueuses par la stimulation de la synthèse du collagène.
L'invention a également pour objet une composition raffermissante comprenant de l'acide cinnamique ou au moins un de ses dérivés.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également constitué de vaisseaux sanguins et de fibres nerveuses. Dans une peau normale, c'est à dire non pathologique ni cicatricielle, le fibroblaste est à l'état quiescent, c'est à dire non prolifératif, peu actif d'un point de vue métabolique et non mobile.

Ce sont les fibres de collagène qui assurent la solidité du derme. Elles sont trés résistantes mais sensibles à certaines enzymes appelées collagénases. Dans le derme, les fibres de collagène sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.
Les fibres de collagènes sont constamment renouvelées mais ce renouvellement diminue avec l'âge ce qui entraîne un amincissement du derme. Mais, cet amincissement du derme est également dû à des causes pathologiques comme par exemple l'hypersécrétion d'hormones corticoïdes, certaines maladies (syndrome de Marfan, syndrome de Ehlers-Danlos) ou encore des carences vitaminiques (scorbut). Il est également admis que des facteurs extrinsèques comme les rayons ultra-violets, le tabac ou certains traitements (Acide rétinoïque et dérivés, glucocorticoïdes, vitamine D et dérivés par exemple) ont également un effet sur la peau et sur son taux de collagène. Une dégradation des fibres de collagène entraîne l'apparence de peau mole et ridée qui est depuis toujours combattue, l'être humain préférant l'apparence d'une peau lisse et tendue.

Par ailleurs, à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.
Il est démontré que les femmes perdent 2,1% de leur taux de collagène par an après la ménopause et que 30% sont perdus dans les cinq premières années postménopausiques.

On comprend donc l'importance que revêt la présence de fibres de collagène dans la peau et l'importance qu'il existe à maintenir, voire renforcer, leur présence.

Il est donc important de pouvoir disposer de produits dont les effets visent à maintenir le taux de collagène dans la peau et lui maintenir une apparence lisse et tendue.

A cet égard la demanderesse a de manière surprenante et inattendue découvert que l'acide cinnamique et ses dérivés présentent la propriété de stimuler la synthèse du collagène.

L'acide cinnamique est présent sous forme trans dans les huiles essentielles de basilic ou de cannelle, dans la balsamine du Pérou ou encore dans les feuilles de cacao. La forme cis est présente dans l'huile d'*Alpinia malacensis*.

Dans l'art antérieur l'acide cinnamique ou ses dérivés sont connus pour être utilisés dans des compositions pour la prévention des escarres (JP 07 242 558), comme actif anti-ultraviolets (US 5 093 109), dans des compositions pour permanente (DE 3 301 515, DE 2 912 427, EP 22 996), dans des lotions capillaires (JP 7 053 401, JP 3 041 413), dans des compositions dépigmentantes (JP 5 221 845, JP 1 186 811), comme anti-oxydant (EP 664 290).

A la connaissance de la demanderesse il n'a jamais été décrit dans l'état de la technique l'utilisation de l'acide cinnamique ou de ses dérivés pour stimuler la synthèse du collagène.

L'invention a donc pour objet l'utilisation dans une composition cosmétique d'une quantité efficace d'acide cinnamique ou d'au moins l'un de ses dérivés, la composition étant destinée à stimuler la synthèse du collagène.

Ainsi, selon l'invention, l'acide cinnamique ou ses dérivés peuvent être d'origine naturelle ou synthétique. Par origine naturelle, on entend l'acide cinnamique ou ses dérivés préparés à partir de matériel végétal dans lequel ils se trouvent présents à l'état naturel. Par origine synthétique, on entend l'acide cinnamique ou ses dérivés préparés par synthèse chimique ou par biotechnologie.
Ainsi, par la suite dans le texte le terme acide cinnamique s'entend comme désignant de l'acide cinnamique ou ses dérivés d'origine naturelle ou synthétique purifiés ou toute préparation les contenant.

Parmi les dérivés d'acide cinnamique utilisables selon l'invention on peut citer à titre d'exemple les esters, les aldéhydes, les alcools, les acides mono- et poly-hydroxycinnamiques et dérivés.

Préférentiellement, selon l'invention on utilise l'acide cinnamique.

Bien entendu, il est possible d'utiliser selon l'invention l'acide cinnamique ou ses dérivés seuls ou en mélange.

Particulièrement l'acide cinnamique ou la composition le contenant sont utilisés selon l'invention en application topique sur la peau et/ou les muqueuses.

On a vu précédemment que le collagène est impliqué dans la solidité du derme, donc dans la fermeté de la peau et/ou des muqueuses.

Ainsi, un des aspects de l'invention est donc de proposer l'utilisation dans une composition cosmétique d'une quantité efficace d'acide cinnamique ou d'au moins un de ses dérivés, l'acide cinnamique ou la composition étant destiné à diminuer les signes cutanés du vieillissement, plus particulièrement diminuer l'apparence de la peau molle et/ou ridée.

Un autre aspect de l'invention est donc de proposer l'utilisation dans une composition cosmétique d'une quantité efficace d'acide cinnamique ou d'au moins un de ses dérivés, l'acide cinnamique ou la composition étant destiné à stimuler le raffermissement de la peau et/ou des muqueuses.

Selon aussi un autre aspect, l'invention a pour objet l'utilisation dans une composition cosmétique d'une quantité efficace d'acide cinnamique ou d'au moins un de ses dérivés, l'acide cinnamique ou la composition étant destiné à favoriser le lissage de la peau et/ou pour tendre la peau.

Selon encore un autre aspect, l'invention a pour objet l'utilisation dans une composition cosmétique d'une quantité efficace d'acide cinnamique ou d'au moins un de ses dérivés, l'acide cinnamique ou la composition étant destiné à combattre les effets cutanés de la ménopause, plus particulièrement les effets de la ménopause sur le collagène.

La quantité d'acide cinnamique utilisable selon l'invention dépend bien évidemment de l'effet recherché et doit être en une quantité efficace pour stimuler la synthèse du collagène.

A titre d'exemple la quantité d'acide cinnamique utilisable selon l'invention peut aller par exemple de 10⁻⁶% à 10% et de préférence de 10⁻³% à 5% du poids total de la composition.

Il est possible d'utiliser dans les compositions de l'invention de l'acide cinnamique en association avec un autre produit stimulant la synthèse du collagène.
Parmi ces autres produits stimulant la synthèse du collagène on peut citer les hormones végétales ou encore la vitamine C ou ses dérivés.

Parmi les hormones végétales on peut citer les auxines telles que l'acide 3-indolacétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile.

Préférentiellement, selon l'invention on utilise l'acide β-naphtoxyacétique.

La peau étant constituée de bien d'autres composants que le collagène, il s'avère intéressant lorsque l'on favorise sa synthèse par de l'acide cinnamique de favoriser en même temps la synthèse de ces autres composants comme par exemple les lipides.

Ainsi, il est possible d'utiliser dans les compositions selon l'invention de l'acide cinnamique ou l'un de ces dérivés en association avec un autre produit stimulant par exemple la synthèse des lipides.

A cet égard les hormones végétales comme les auxines et particulièrement l'acide β-naphtoacétique peuvent encore être citées.

Ainsi, l'invention a pour objet une composition cosmétique comprenant de l'acide cinnamique ou l'un de ses dérivés et au moins un autre produit stimulant la synthèse des lipides.

L'invention a en outre pour objet une composition cosmétique raffermissante comprenant dans un milieu cosmétiquement acceptable une quantité efficace d'acide cinnamique ou d'au moins un de ses dérivés.

L'invention a également pour objet une composition cosmétique pour le lissage de la peau ou pour tendre la peau comprenant dans un milieu cosmétiquement acceptable une quantité efficace d'acide cinnamique ou d'au moins un de ses dérivés.

L'invention a encore pour objet une composition cosmétique pour stimuler la synthèse du collagène comprenant dans un milieu cosmétiquement acceptable une quantité efficace d'acide cinnamique ou d'au moins un de ses dérivés.

L'invention a également pour objet une composition cosmétique pour combattre les effets cutanés de la ménopause comprenant dans un milieu cosmétiquement acceptable une quantité efficace d'acide cinnamique ou d'au moins un de ses dérivés.

L'invention a enfin pour objet une composition cosmétique pour combattre les effets de la ménopause sur le collagène comprenant dans un milieu cosmétiquement acceptable une quantité efficace d'acide cinnamique ou d'au moins un de ses dérivés.

Par milieu cosmétiquement acceptable on entend un milieu compatible avec la peau, les muqueuses, les ongles, les cheveux.

En outre, l'invention a pour objet des compositions comprenant en plus de l'acide cinnamique ou l'un de ses dérivés, un autre composé ayant la propriété de stimuler la synthèse du collagène comme en particulier les hormones végétales (auxines par exemple) ou la vitamine C et ses dérivés.

L'invention a aussi pour objet des compositions comprenant en plus de l'acide cinnamique ou l'un de ses dérivés, un composé ayant la propriété de stimuler la synthèse des lipides, particulièrement de lipides totaux de la peau.

La composition selon l'invention comprend bien évidemment un support cosmétiquement acceptable et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser selon l'invention en association avec l'acide cinnamique ou l'un au moins de ses dérivés, des composés choisis parmi
- les hormones végétales ;
- les agents antibactériens tels que les macrolides, les pyranosides et les tétracyclines, et notamment l'érythromycine ;
- les agents antagonistes de calcium, comme le vérapamil et le diltiazem ;
- des capteurs de radicaux OH, tels que le diméthylsulfoxyde ;
- des extraits de végétaux tels que ceux d'Iridacés ou de soja, extraits pouvant alors contenir ou non des isoflavones ;
- des extraits de micro-organismes dont en particulier des extraits bactériens comme ceux de bactéries filamenteuses non photosynthétiques .

A la liste ci-dessus, d'autres composés peuvent également être ajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétraénoïque et eicosatriénoïque ou leurs esters et amides, la vitamine D et ses dérivés.

Selon l'invention, on peut, entre autres, associer l'acide cinnamique à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les agents modulant l'adhésion bactérienne sur la peau et /ou les muqueuses tels que le miel, notamment le mile d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase, composés décrits comme étant actifs dans le traitement des peaux sensibles et comme présentant des effets anti-irritants, en particulier vis-à-vis de composés irritants éventuellement présents dans les compositions.

Ainsi, un autre objet de l'invention concerne une composition comprenant une quantité efficace de l'acide cinnamique ou de l'un de ses dérivés et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques, les agents modulant la différenciation et/ou la prolifération et/ou la pigmentation cutanée, les antagonistes de substance P, de CGRP ou de bradykinine ou les inhibiteurs de NO synthase.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

Bien entendu, l'acide cinnamique, ou ses dérivés, peut être utilisé dans la préparation de compositions cosmétiques et/ou pharmaceutiques, particulièrement dermatologiques, destinées à stimuler la synthèse du collagène.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1 : Etude de l'effet de l'acide cinnamique sur la synthèse du collagène.

L'étude est faite par mesure de l'incorporation de proline radioactive dans des cultures de fibroblastes dermiques humains normaux.
Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 UI/ml) et de 10% de sérum de veau foetal (Gibco).

Le test est effectué sur des cultures de cellules à 80% de confluence en plaque de 24 trous. L'acide cinnamique, à la concentration de 10⁻⁴M, est mis en contact avec les cellules pendant 48 heures. Le marquage à la proline tritiée (L-[2,3-³H]-proline vendue par Amersham, 33 µCi/ml) est effectué pendant 24 heures.
Le taux de proline tritiée incorporée est mesuré en fin de test par précipitation acide des protéines sur filtres et comptage en scintillation liquide.

Les résultats sont évalués par rapport à un témoin constitué par des cellules n'ayant pas été traitées à l'acide cinnamique.

Un témoin positif (vitamine C à 20µg/ml) connue pour stimuler la synthèse du collagène et un témoin négatif (l'acide rétinoïque à 10⁻⁶M) connu pour inhiber la synthèse du collagène sont introduits dans le test comme référence.

Les résultats de ce test sont présentés dans le tableau suivant.

| Traitement | cpm | % | p |
|---|---|---|---|
| Cellules non traitées | 19261 | 100 | - |
| Acide cinnamique | 26209 | 136 | <0,01 |
| Acide rétinoïque | 13160 | 68 | <0,01 |
| Vitamine C | 40344 | 209 | <0,01 |

| | | | |
|---|---|---|---|
| cpm : coups par minute | | | |
| p : intervalle de confiance calculé selon la méthode de Dunett. | | | |

Ces résultats montrent que l'acide cinnamique stimule de façon significative l'incorporation de proline dans le collagène et donc qu'il présente un effet sur la néosynthèse du collagène.

### Exemple 2 : Exemples de compositions selon l'invention. Ces compositions sont obtenues par les techniques habituelles couramment utilisées en cosmétique ou en pharmacie.

### Composition 1 : crème de soin

| | | |
|---|---|---|
| Cire d'abeille | 1,5 | % |
| Huile d'amandes d'abricot | 13,0 | % |
| Conservateurs | 0,3 | % |
| Parfum | 0,4 | % |
| Acide β-naphtoxyacétique | 0,01 | % |
| Acide cinnamique | 0,01 | % |
| Xanthane | 0,5 | % |
| Cyclopenta diméthylsiloxane | 5,0 | % |
| Eau déminéralisée stérilisée | 69,28 | % |
| Mono-di-palmitostéarate de sucrose | 3,0 | % |
| Sesquistéarate de méthylglucose | 3,0 | % |
| Acide stéarique | 1,0 | % |
| Alcool cétylique | 3,0 | % |

### Composition 2 : huile corporelle

| | | |
|---|---|---|
| Huile de vaseline | 47,98 | % |
| Huile d'amandes d'abricot | 6,0 | % |
| Parfum | 1,0 | % |
| Acide β-naphtoxyacétique | 0,01 | % |
| Acide cinnamique | 0,01 | % |
| Cyclopenta diméthylsiloxane | 45,0 | % |

### Composition 3 : lait démaquillant

| | | |
|---|---|---|
| Palmitate d'éthyl-2 hexyle | 10,5 | % |
| Fraction liquide de beurre de karité | 16,5 | % |
| Conservateurs | 0,3 | % |
| Parfum | 0,15 | % |
| Acide β-naphtoxyacétique | 0,01 | % |
| Acide cinnamique | 0,01 | % |
| Hydroxyde de sodium | 0,04 | % |
| Polymère carboxyvinylique | 0,2 | % |
| Eau déminéralisée stérilisée | 69,79 | % |
| Mélange de cétylstéarylglucoside | | |
| et d'alcools cétylique et stéarylique | 2,5 | % |

### Composition 4 : crème de soin

| | | |
|---|---|---|
| Cire d'abeille | 1,5 | % |
| Huile d'amandes d'abricot | 13,0 | % |
| Conservateurs | 0,3 | % |
| Parfum | 0,4 | % |
| Acide β-naphtoxyacétique | 0,01 | % |
| Acide cinnamique | 0,01 | % |
| Cinnamate d'éthyle | 0,01 | % |
| Xanthane | 0,5 | % |
| Cyclopenta diméthylsiloxane | 5,0 | % |
| Eau déminéralisée stérilisée | 69,27 | % |
| Mono-di-palmitostéarate de sucrose | 3,0 | % |
| Sesquistéarate de méthylglucose | 3,0 | % |
| Acide stéarique | 1,0 | % |
| Alcool cétylique | 3,0 | % |

## Revendications

1. Utilisation dans une composition cosmétique d'une quantité efficace d'au moins un dérivé d'acide cinnamique comme agent destiné à stimuler la synthèse de collagène.

2. Utilisation selon la revendication 1, **caractérisée par le fait que** le dérivé d'acide cinnamique est destiné à stimuler le raffermissement de la peau et/ou des muqueuses.

3. Utilisation selon la revendication 1, **caractérisée par le fait que** le dérivé d'acide cinnamique est destiné à combattre les effets de la ménopause sur le collagène.

4. Utilisation selon la revendication 1, **caractérisée par le fait que** le dérivé d'acide cinnamique est destiné à tendre la peau.

5. Utilisation selon l'une quelconque des revendications précédentes, **caractérisée par le fait que** le dérivé d'acide cinnamique est choisi parmi les esters, les aldéhydes, les alcools, les acides mono- et poly-hydroxycinnamiques et dérivés.

6. Utilisation dans une composition cosmétique d'une quantité efficace d'au moins un dérivé d'acide cinnamique choisi parmi les esters, les alcools, les aldéhydes et leurs dérivés, **caractérisée par le fait que** le dérivé d'acide cinnamique est destiné à diminuer les signes cutanés du vieillissement.

7. Utilisation dans une composition cosmétique d'une quantité efficace d'au moins un dérivé d'acide cinnamique choisi parmi les esters, les alcools, les aldéhydes et leurs dérivés, **caractérisée par le fait que** le dérivé d'acide cinnamique est destiné à favoriser le lissage de la peau.

8. Utilisation selon l'une des revendications 5 à 7, **caractérisée par le fait que** l'ester d'acide cinnamique est le cinnamate d'éthyle.

9. Utilisation selon l'une quelconque des revendications précédentes, **caractérisée par le fait que** la composition est utilisée en application topique sur la peau et/ou les muqueuses.

10. Utilisation selon l'une quelconque des revendications précédentes, **caractérisée par le fait que** le dérivé d'acide cinnamique est présent en une quantité allant de 10⁻⁶% à 10% du poids total de la composition.

11. Utilisation selon l'une quelconque des revendications précédentes, **caractérisée par le fait que** le dérivé d'acide cinnamique est présent en une quantité allant de 10⁻³% à 5% du poids total de la composition.

12. Utilisation selon l'une quelconque des revendications précédentes, **caractérisée par le fait que** le dérivé d'acide cinnamique est associé à un autre produit stimulant la synthèse du collagène.

13. Utilisation selon la revendication précédente, **caractérisée par le fait que** l'autre produit stimulant la synthèse du collagène est choisi parmi les hormones végétales et ou la vitamine C ou ses dérivés.

14. Utilisation selon la revendication précédente, **caractérisée par le fait que** l'hormone végétale est une auxine.

15. Utilisation selon la revendication précédente, **caractérisée par le fait que** l'auxine est choisie parmi l'acide indole-3-acétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'indole acétaldéhyde et l'indole acétonitrile.

16. Utilisation selon la revendication précédente, **caractérisée par le fait que** l'auxine est de l'acide β-naphtoxyacétique.

17. Utilisation selon l'une quelconque des revendications précédentes, **caractérisée par le fait que** le dérivé d'acide cinnamique est associé à un autre produit stimulant la synthèse des lipides.

18. Utilisation selon la revendication précédente, **caractérisée par le fait que** le produit stimulant la synthèse des lipides est une hormone végétale telle que définie dans les revendications 14 à 16.

19. Utilisation selon l'une quelconque des revendications 12 à 18, **caractérisée par le fait que** l'autre produit stimulant la synthèse du collagène et/ou des lipides est en une quantité comprise entre 10⁻⁶% à 10%, et de préférence entre 10⁻³% à 5% du poids total de la composition.

20. Composition cosmétique comprenant dans un milieu cosmétiquement acceptable au moins une quantité efficace d'au moins un dérivé d'acide cinnamique et au moins un autre produit stimulant la synthèse du collagène.

21. Composition selon la revendication précédente, **caractérisée par le fait que** le produit stimulant la synthèse du collagène est choisi parmi les hormones végétales ou la vitamine C et ses dérivés.

22. Composition cosmétique comprenant dans un milieu cosmétiquement acceptable au moins une quantité efficace d'au moins un dérivé d'acide cinnamique et un composé stimulant la synthèse des lipides.

23. Composition selon la revendication précédente, **caractérisée par le fait que** le composé stimulant la synthèse des lipides est une hormone végétale.
